# EUROPEAN PATENT APPLICATION

(11) **EP 4 578 443 A1**
(43) Date of publication of application: **02.07.2025**
(21) Application number: 23386142.6
(22) Date of filing: 27.12.2023
(51) Int. Cl.: A61K 9/10, A61K 31/496, A61K 47/14

(54) **ORAL SUSPENSIONS COMPRISING NINTEDANIB ESYLATE**

(71) Applicant: Faran S.A., 14564 Nea Kifissia (GR)
(72) Inventor: Koukoulommatis, Panagiotis, 15238 Chalandri (GR); Panagiotopoulos, Tsampikos-Dimitrios, 15124 Marousi (GR); Tziala, Soultana, 19016 Artemida (GR)
(74) Representative: Roukounas, Dimitrios

(57) **Abstract**

Oral pharmaceutical suspension comprising nintedanib esylate and a non-aqueous liquid carrier comprising one or more medium-chain fatty acid triglycerides and at least one suspending agent.

## Description

### FIELD OF THE INVENTION

The present invention relates to oral pharmaceutical suspensions comprising as active substance nintedanib esylate.

### BACKGROUND OF THE INVENTION

Nintedanib is a triple angiokinase inhibitor that blocks the kinase activity of vascular endothelial growth factor receptors (VEGFR 1-3), platelet-derived growth factor receptors (PDGFR -α and -β), and fibroblast growth factor (FGFR 1-3). Nintedanib competitively binds to the adenosine triphosphate (ATP) binding site of these receptors and blocks intracellular signaling that is essential for the proliferation and survival of endothelial as well as perivascular cells (pericytes and vascular smooth muscle cells).

Nintedanib, was first disclosed in US6762180.

Nintedanib esylate is a yellow, crystalline solid that melts at 244°C to 251°C. It has little solubility in water and somewhat better solubility in dimethyl sulfoxide (DMSO) at 25 g/l.

Nintedanib esylate, was first disclosed in US7119093.

Nintedanib esylate is currently commercially available as 100 mg and 500 mg soft capsules under the brand name Ofev^{®} (or Vargatef^{®}).

Although oral solid dosage forms such as tablets are very popular, mainly due to ease of management, for certain users (e.g. children) these forms are not necessarily a convenient option, especially due to difficulty in swallowing these forms. This lack of convenience results in high incidence of non-compliance and ineffective therapy.

Moreover, the Patient Information Leaflet (PIL) of Ofev^{®} discloses a dosing scheme according to which the recommended dose of nintedanib esylate is 150 mg twice daily approximately 12 hours apart, taken with food. However, the recommended dosage may be varied in patients with mild hepatic impairment (Child Pugh A) as 100 mg twice daily approximately 12 hours apart, taken with food. In the event of management of adverse reactions, temporary dose reduction to 100 mg or discontinuation of the composition is employed.

This dosing scheme, as disclosed in the PIL of Ofev^{®} sets as prerequisite pharmaceutical forms, such as oral liquid suspensions, that enable dose fractioning.

Drugs are formulated as suspensions for different reasons, but the most common one is low solubility. However, it is apparently extremely difficult to formulate nintedanib esylate in the form of an aqueous suspension for oral administration, as it is unstable at least in contact with water.

WO2022/079737, reports that both nintedanib and nintedanib esylate have low solubility and stability under neutral conditions.

Furthermore, a suspension, unlike a syrup in which the drug is fully dissolved, requires adequate shaking of the container to resuspend the drug uniformly before dosing. Difficult redispersion of the drug from a sediment, or in the worst case, from caking, will result in under- and overdosing. This problem of variable dosing is also encountered when the patient or the caregiver forgets to shake the container before dosing. It is therefore desirable to produce a suspension that is able to maintain its homogeneity on prolonged storage without vigorous shaking.

Hence, there is still the need for an oral pharmaceutical suspension, comprising nintedanib esylate, which exhibits excellent physicochemical stability and remain homogeneous during prolonged period of time without vigorous shaking.

### SUMMARY OF INVENTION

The present invention provides a physiochemically stable oral pharmaceutical suspension comprising nintedanib esylate that remains homogeneous for prolonged period of time without vigorous shaking.

The oral pharmaceutical suspension according to the invention comprises nintedanib and a non-aqueous liquid carrier comprising one or more medium-chain fatty acid triglycerides and at least one suspending agent.

The oral pharmaceutical suspension according to the invention also exhibits excellent physicochemical stability.

### DETAILED DESCRIPTION OF THE INVENTION

The present invention provides an oral pharmaceutical suspension comprising nintedanib esylate, in association with a pharmaceutically acceptable non-aqueous liquid carrier.

The term "% w/v" used in the present description and claims refers to g of the respective substance per 100 ml of the oral suspension.

As used throughout the present description and claims, the term "non-aqueous" means essentially water-free.

As used throughout the present description and claims, the term "suspending agent" means an ingredient that promotes particle suspension or dispersion and reduces sedimentation.

There are numerous lipids comprising triglycerides of fatty acids available to formulators as excipients for lipid-based drug delivery systems. Many synthetic lipids are also available in which the glycerol backbone has been replaced by propylene glycol and/or polyethylene glycols. Additionally, the degree of esterification of the fatty acid moiety may vary, forming mono-, di- and tri-glycerides as well as different esters of propylene glycol and polyethylene glycols. The fatty acids are not necessarily long chain (C₁₁-C₂₂); they can be medium-chain (C₆-C₁₀), short chain, unsaturated or branched. Due to these differences in chemical nature, there are numerous lipids or lipid-like excipients available commercially, all of which are colloquially called 'lipids' in the pharmaceutical field.

The term "medium-chain triglycerides" according to the present invention refers to triglycerides of saturated straight chain fatty acids having a chain of 6 to 10 carbon atoms (C_{6:0} to C_{10:0}).

It has been found that many lipophilic surfactants, commonly used in drug carrier systems such as polysorbate 80, polyethylene glycol castor oils and polyethylene glycol hydrogenated castor oils are ineffective to slow down the decomposition nintedanib esylate after storage.

On the other hand, it has now been found that the physicochemical stability of nintedanib esylate is considerably enhanced in non-aqueous liquid carriers comprising one or more medium-chain fatty acid triglycerides, especially at concentrations of at least 45% w/v. This finding is unexpected since it does not apply to medium-chain fatty acid monoglycerides or diglycerides. In addition, this finding does not apply to other triglycerides, such as long chain triglycerides.

Furthermore, it has been found that the use of xanthan gum as suspending agent leads to non-sufficient dispersibility of nintedanib esylate particles in the oral suspension.

In contrast, it has been found that the use of certain other suspending agents results in sufficient dispersion of the nintedanib esylate particles in the oral suspension without the need for vigorous shaking of the oral suspension.

The oral pharmaceutical suspension according to the invention comprises nintedanib esylate and a non-aqueous liquid carrier comprising one or more medium-chain fatty acid triglycerides and at least one suspending agent selected form the group consisting of colloidal silicon dioxide, bentonite, potassium alginate, and a mixture of microcrystalline cellulose and sodium carboxymethylcellulose. Preferably the suspending agent is selected form the group consisting of colloidal silicon dioxide, potassium alginate and a mixture of microcrystalline cellulose and sodium carboxymethylcellulose. More preferably the suspending agent is colloidal silicon dioxide.

Furthermore, the mixture of microcrystalline cellulose and sodium carboxymethylcellulose has 8.3 to 13.8 % w/w sodium carboxymethylcellulose content. Examples of commercially available mixtures of microcrystalline cellulose and sodium carboxymethylcellulose that can be used as suspending agents in the present invention include VIVAPUR^{®} MCG 581 P and VIVAPUR^{®} MCG 591 P.

Preferably, the oral pharmaceutical suspension according to the invention comprises from 2.0 % w/v to 10 % w/v nintedanib esylate.

More preferably, the oral pharmaceutical suspension according to the invention comprises from 4.0 % w/v to 8 % w/v nintedanib esylate.

Preferably, the total concentration of suspending agents in the oral pharmaceutical suspension according to the invention is from 0.5% w/v to 10% w/v.

More preferably, the total concentration suspending agents is from 1% w/v to 5% w/v.

The European Pharmacopoeia (Ph. Eur. 6.0) describes medium-chain triglycerides as the fixed oil extracted from the hard, dried fraction of the endosperm of Cocos nucifera L. or from the dried endosperm of Elaeis guineenis Jacq. They comprise a mixture of triglycerides of saturated fatty acids, mainly of caprylic acid (C_{8:0}) and of capric acid (C_{10:0}). They contain no less than 95% w/w of saturated fatty acids (expressed as percent (%) by weight of total mixture).

According to Handbook of excipients, sixth edition (2009), medium-chain triglycerides (synonyms; MCT oil, caprylic/capric triglyceride, glyceryl tricaprylate/caprate) may also be known as fractionated coconut oil, which contains three saturated lipid chains bound to a glycerin backbone, and are distinguished from other triglycerides by the length of the carbon chains, normally from 6 to 10.

Medium chain triglycerides have been used in a variety of pharmaceutical formulations including oral, parenteral and topical preparations mainly as emulsifying agents or solvents.

According to a preferred embodiment of the invention, the medium-chain triglyceride is caprylic triglyceride.

According to another preferred embodiment of the invention, the medium-chain triglyceride is a mixture of caprylic and capric triglycerides. Preferably, the medium-chain triglyceride according to the invention is a mixture of caprylic and capric triglycerides in a weight ratio caprylic acid : capric acid from 4:6 to 99:1. More preferably, the medium-chain triglyceride according to the invention is a mixture of caprylic and capric triglycerides in a weight ratio caprylic acid : capric acid from 4.5:5.5 to 95:5. Even more preferably, the medium-chain triglyceride according to the invention is a mixture of caprylic and capric triglycerides in a weight ratio caprylic acid : capric acid from 5:5 to 90:10.

Preferably the medium-chain triglyceride according to the invention has a saponification value from 320 mg KOH/g to 380 mg KOH/g. More preferably the medium-chain triglyceride according to the invention has a saponification value from 330 mg KOH/g to 370 mg KOH/g. Even more preferably the medium-chain triglyceride according to the invention has a saponification value from 335 mg KOH/g to 365 mg KOH/g.

Preferable examples of medium-chain triglycerides according to the invention include
∘ caprylic triglyceride with fatty-acid composition of about 99% w/w caprylic acid (C_{8:0}) (synonyms: tricaprylin, glycerol trioctanoate), with a saponification value of 335 mg KOH/g to 360 mg KOH/g e.g. commercially available Captex^{®} 8000, which is a synthetic triglyceride manufactured by esterification of caprylic acid and glycerin.
∘ caprylic/capric triglyceride with fatty-acid composition of 65% w/w to 80% w/w caprylic acid (C_{8:0}), 20% w/w to 35% w/w capric acid, less than 2% w/w caproic acid (C_{6:0}), less than 2% w/w lauric acid (C_{12:0}) and less than 1% w/w myristic acid (C_{14:0}), with a saponification value of 335 mg KOH/g to 355 mg KOH/g e.g. commercially available Miglyol^{®} 810, BergaBest^{®} MCT Oil 70/30 & Captex^{®} 300.
∘ caprylic/capric triglyceride with fatty-acid composition of 55% w/w to 70% w/w caprylic acid (C_{8:0}) & 30% w/w to 45% w/w capric acid (C_{10:0}), with a saponification value of 330 mg KOH/g to 360 mg KOH/g. e.g. commercially available Neobee^{®} M-5, Cromadol^{®} GTCC & Myritol^{®} 318.
∘ caprylic/capric triglyceride with fatty-acid composition of 50 w/w to 65% w/w caprylic acid (C_{8:0}), 30% w/w to 45% w/w capric acid (C_{10:0}), less than 2% w/w caproic acid (C_{6:0}), less than 2% w/w lauric acid (C_{12:0}) and less than 1% w/w myristic acid (C_{14:0}), with a saponification value of 325 mg KOH/g to 345 mg KOH/g e.g. commercially available Miglyol^{®} 812, BergaBest ^{®} MCT Oil 60/40 & Captex^{®} 355.

Preferably, the total concentration of the medium-chain triglycerides in the oral pharmaceutical suspension according to the invention is from 45% w/v to 95% w/v.

More preferably, the total concentration of the medium-chain triglycerides is from 50% w/v to 90% w/v.

The oral pharmaceutical suspension, according to the invention may also comprise additional excipients commonly used in preparing oral liquid compositions, such as antimicrobial preservatives, antioxidants, sweeteners and flavouring agents.

Antimicrobial preservatives may include but are not limited to sodium benzoate, benzoic acid, boric acid, sorbic acid and their salts thereof, benzyl alcohol, parahydroxybenzoic acids and their alkyl esters, methyl, ethyl and propyl parahydroxybenzoates and their salts or mixtures thereof.

Antioxidants which may be used in the present invention comprise, amongst others, butylated hydroxytoluene, butylated hydroxyanisole, ethylenediamine tetraacetic acid ("EDTA"), ascorbic acid, sodium metabisulfite and propyl gallate or any combinations thereof.

Sweeteners may include but are not limited to aspartame, acesulfame potassium, thaumatin, saccharin and salts thereof, sodium cyclamate, glycyrrhizin, monosodium glycyrrhizinate, monoamonium glycyrrhizinate or mixtures thereof.

The oral pharmaceutical suspension, according to the invention may further comprise flavours and/or colours so as to enhance its palatability and/or visual appearance. Suitable flavouring agents and colouring agents are well known to those skilled in the art. The flavouring agent may be a natural or artificial flavouring agent, including an essence, an extract, a flavour oil or combinations thereof. Exemplary flavours include, but are not limited to: honey flavour, raspberry flavour, strawberry flavour, blueberry flavour, blackberry flavour, grape flavour, peach flavour, apricot flavour, watermelon flavour, melon flavour, fruit punch flavour, cranberry flavour, mango flavour, banana flavour, citrus flavour, orange flavour, lemon flavour, grapefruit flavour, cherry flavour, vanilla flavour, caramel flavour, chocolate flavour, marshmallow flavour, coffee flavour and coconut flavour.

The oral pharmaceutical suspension according to the invention is preferably supplied as multidose preparation. Each dose from a multidose container may be administered by means of a device suitable for accurately measuring the prescribed volume. The device is usually a spoon or a cup for volumes of 5 ml or multiples thereof, or an oral syringe for other volumes. Preferably, the device is an oral syringe.

The oral pharmaceutical suspension of the present invention may be prepared using methods well known in the art and using regular manufacturing equipment.

For example, it may be prepared using the following process: The active substance and the excipients are weighed. The selected medium-chain triglyceride(s) is added into a vessel and heated to 30 - 35°C. The selected suspending agent(s) is added into the vessel under continuous stirring. Nintedanib esylate is added into the vessel under stirring. The remaining excipients if present, are successively added under continuous stirring. Finally, the volume is adjusted with a quantity of the medium-chain triglyceride(s).

The final suspension is optionally filtered over a 10 µm filter, and filled preferably in light-protective containers, such as amber type III glass 50 or 100 ml bottles sealed with child resistant, tamper evident screw caps.

### EXAMPLES

### EXAMPLE 1

These nintedanib esylate suspensions were prepared in the following manner;

Approximately 80% of the total quantity of the selected medium-chain triglycerides, oils or glyceride mixtures was added into a vessel and heated to 30°C - 35°C. Nintedanib esylate was added into the vessel under stirring. The selected suspending agent was then added into the vessel under continuous stirring. The remaining quantity of the selected medium-chain triglyceride, oils or glyceride mixtures were then added under continuous stirring. Finally, the volume was adjusted with the required quantity of the above medium-chain triglycerides, oils or glyceride mixtures.

In order to test the uniformity of the suspensions the following method was applied: A 65 ml bottle was filled with 60 ml of each of the prepared suspensions and stored for three months at room temperature (20°C to 30°C). After 10 seconds of hand shaking of the bottle, five 10 ml fractions were then carefully withdrawn from the surface of the liquid with a digital pipette and analyzed separately. The remaining 10 ml in the bottle, representing the sixth fraction, were also analyzed. Quantification of nintedanib esylate in each of the six fractions was performed by HPLC. The results presented below show the range of nintedanib esylate content in the six fractions as percentage of the label claim, i.e., of the theoretically calculated content of each fraction. Furthermore, the results show the relative standard deviation of the measurements.

**Table 1**

| | **Composition** | | |
|---|---|---|---|
| | **I** | **II** | **III** |
| **Active substance** | **% w/v** | | |
| Nintedanib esylate | 6.2 | 6.2 | 6.2 |

| **Excipients** | **% w/v** | | |
|---|---|---|---|
| Miglyol 810 N^{®} (65 to 80% caprylic (C8:0) / 20% to 35% capric (C10:0) triglyceride) | 92.4 | 92.4 | 92.4 |
| Xanthan gum | 1.0 | - | - |
| Colloidal silicon dioxide | - | 1.0 | 2.0 |
| Lemon flavor | 0.1 | 0.1 | 0.1 |
| Sucralose | 0.3 | 0.3 | 0.3 |

| Uniformity test results (90 days at room temperature) | | | |
|---|---|---|---|
| Range of six fractions (% label claim) | 85 - 117 | 91 - 109 | 92 - 108 |
| Relative Standard Deviation | 11.6% | 6.8% | 6.3% |

The uniformity test results of table 1 show that the use of xanthan gum as suspending agent does not maintain the content uniformity of nintedanib esylate at desirable levels.

The compositions were stored at a temperature of 40°C and relative humidity of 75% for a period of three months. Quantification of nintedanib esylate's degradation impurities was performed by HPLC.

**Table 2**

| | **40°C± 2°C, 75% RH** | |
|---|---|---|
| | **T** = **0** | **T** = **3 months** |
| Trial I | Total impurities: < 0.1% | Total impurities: < 0.1% |
| Trial II | Total impurities: < 0.1% | Total impurities: < 0.1% |
| Trial III | Total impurities: < 0.1% | Total impurities: < 0.1% |

The stability results of table 2 show that the selection of the suspending agent does not affect the stability profile of the nintedanib esylate oral suspensions when the non-aqueous liquid carrier comprises a medium chain triglyceride according to the invention.

### EXAMPLE 2

Table 3 shows compositions of oral suspensions according to the present invention.

These nintedanib esylate suspensions were prepared in the following manner;

Approximately 80% of the total quantity of the selected medium-chain triglycerides, oils or glyceride mixtures was added into a vessel and heated to 30°C - 35°C. Nintedanib esylate was added into the vessel under stirring. The selected suspending agent was then added into the vessel under continuous stirring. The remaining quantity of the selected medium-chain triglyceride, oils or glyceride mixtures were then added under continuous stirring. Finally, the volume was adjusted with the required quantity of the above medium-chain triglycerides, oils or glyceride mixtures.

In order to test the uniformity of the suspensions the following method was applied: A 65 ml bottle was filled with 60 ml of each of the prepared suspensions and stored for three months at room temperature (20°C to 30°C). After 10 seconds of hand shaking of the bottle, five 10 ml fractions were then carefully withdrawn from the surface of the liquid with a digital pipette and analyzed separately. The remaining 10 ml in the bottle, representing the sixth fraction, were also analyzed. Quantification of nintedanib esylate in each of the six fractions was performed by HPLC. The results presented below show the range of nintedanib esylate content in the six fractions as percentage of the label claim, i.e., of the theoretically calculated content of each fraction. Furthermore, the results show the relative standard deviation of the measurements.

**Table 3**

| | **Composition** | | |
|---|---|---|---|
| | **IV** | **V** | **VI** |
| **Active substance** | **% w/v** | | |
| Nintedanib esylate | 6.2 | 6.2 | 6.2 |

| **Excipients** | **% w/v** | | |
|---|---|---|---|
| Miglyol 812 ^{®} (50 to 65% caprylic (C_{8:0}) / 30% to 45% capric (C_{10:0}) triglyceride) | 46.4 | 92.8 | 92.8 |
| Miglyol 810 ^{®} (65 to 80% caprylic (C_{8:0}) / 20% to 35% capric (C_{10:0}) triglyceride) | 46.4 | - | - |
| Potassium alginate | - | 1.0 | - |
| Vivapur^{®} MCG 591P (Microcrystalline cellulose / carboxymethylcellulose sodium) | - | - | 1.0 |

| Uniformity test results (90 days at room temperature) | | | |
|---|---|---|---|
| Range of six fractions (% label claim) | 92 - 108 | 91 - 111 | 90 - 108 |
| Relative Standard Deviation | 6.7% | 7.2% | 6.5% |

The uniformity test results of Table 3 clearly show that the use of certain suspending agents (potassium alginate and microcrystalline cellulose / carboxymethylcellulose sodium) leads to acceptable redispersability of nintedanib esylate particles in the oral suspension when the non-aqueous liquid carrier comprises a medium chain triglyceride according to the invention.

## Claims

1. Oral pharmaceutical suspension comprising nintedanib esylate as active ingredient and a non-aqueous liquid carrier comprising one or more triglycerides of saturated fatty acid having a straight chain of 6 to 10 carbon atoms (medium-chain triglycerides) and at least one suspending agent selected form the group consisting of colloidal silicon dioxide, bentonite, potassium alginate, and a mixture of microcrystalline cellulose and sodium carboxymethylcellulose.

2. Oral pharmaceutical suspension according to claim 1, comprising from 2.0% w/v to 10% w/v nintedanib esylate.

3. Oral pharmaceutical suspension according to claim 1 or 2, comprising from 3% w/v to 8% w/v nintedanib esylate.

4. Oral pharmaceutical suspension according to any one of the preceding claims, wherein the total concentration of medium-chain triglycerides in the suspension is from 45% w/v to 95% w/v.

5. Oral pharmaceutical suspension according to any one of the preceding claims, wherein the total concentration of medium-chain triglycerides in the suspension is from 50% w/v to 90% w/v.

6. Oral pharmaceutical suspension according to any one of the preceding claims, wherein the one or more medium-chain triglycerides is caprylic triglyceride.

7. Oral pharmaceutical suspension according to any one of the preceding claims, wherein the one or more medium-chain triglycerides is a mixture of caprylic and capric triglycerides in a weight ratio caprylic acid : capric acid from 4:6 to 99:1.

8. Oral pharmaceutical suspension according to any one of the preceding claims, wherein the one or more suspending agents is colloidal silicon dioxide.

9. Oral pharmaceutical suspension according to any one of the preceding claims, wherein the total concentration of suspending agents in the suspension is from 0.5% w/v to 10% w/v.

10. Oral pharmaceutical suspension according to any one of the preceding claims, wherein the total concentration of suspending agents in the suspension is from 1% w/v to 5% w/v.

11. Oral pharmaceutical suspension according to any one of the preceding claims, wherein the one or more medium-chain triglycerides have a saponification value from 320 mg KOH/g to 380 mg KOH/g.
